# EUROPÄISCHE PATENTSCHRIFT

(11) **EP 2 905 045 B1**
(45) Veröffentlichungstag und Bekanntmachung des Hinweises auf die Patenterteilung: **23.08.2017**
(21) Anmeldenummer: 15156557.9
(22) Anmeldetag: 18.05.2006
(51) Int. Cl.: A61M 11/00, A61M 15/00, A61M 16/08

(54) **INHALATIONSTHERAPIEVORRICHTUNG**
INHALATION THERAPY DEVICE
DISPOSITIF DE THÉRAPIE PAR INHALATION

(30) Priorität: 24.06.2005 DE 102005029498
(43) Veröffentlichungstag der Anmeldung: 12.08.2015
(62) Teilanmeldung aus: 06010290.2
(73) Patentinhaber: PARI Pharma GmbH, 82319 Starnberg (DE)
(72) Erfinder: Hetzer, Uwe, 81476 München (DE); Gallem, Thomas, 81371 München (DE); Waldner, Robert, 86971 Peiting (DE)
(74) Vertreter: Hoffmann Eitle

(56) Entgegenhaltungen:
- EP-A1- 0 504 459
- EP-A2- 1 023 911
- WO-A1-02/04054
- DE-A1- 10 320 143
- DE-U1- 20 100 648
- US-A- 4 951 661

## Beschreibung

Die vorliegende Erfindung betrifft Inhalationstherapievorrichtungen, mit denen einem Patienten ein Medikament in Form eines Aerosols zur Einatmung bereitgestellt wird.

Mit Inhalationstherapievorrichtungen werden Aerosole für therapeutische Zwecke erzeugt, die hohen Anforderungen gerecht werden müssen. Die Anforderungen ergeben sich aus der Therapie, die mit der Inhalationstherapievorrichtung durchgeführt werden soll. Das Kernstück einer Inhalationstherapievorrichtung ist der Aerosolerzeuger, der aus einem Medikament, das regelmäßig in Form einer flüssigen Formulierung oder eines anderen Fluids vorliegt, ein Aerosol erzeugt, wenn er von einer in der Inhalationstherapievorrichtung vorhandenen Steuereinheit zur Aerosolerzeugung angesteuert wird. Der Aerosolerzeuger weist in einer vorteilhaften Ausgestaltung zumindest eine Membran und einen Schwingungserzeuger auf, wobei die Membran von dem Schwingungserzeuger in Schwingungen versetzt werden kann und dann aus dem an die eine Seite der Membran zugeführten Medikament ein Aerosol erzeugt, das auf der anderen Seite der Membran abgegeben wird. Die entstehende Aerosolwolke dehnt sich in einem vor dem Aerosolerzeuger befindlichen Raumbereich aus, der regelmäßig als eine Kammer in dem Gehäuse des Inhalationstherapiegeräts realisiert ist. Um Medikamentverluste zu minimieren, sind Form und Größe der Kammer vorteilhafterweise so ausgelegt, däss sich möglichst wenige Aerosoltröpfchen/-partikel an der Wand der Kammer niederschlagen.

Der Einsatz von Inhalationstherapiegeräten im Zusammenhang mit Beatmungsgeräten, bei denen ein Patient beatmet wird oder über die zugeführte Beatmungsluft ein Atemmuster vorgegeben erhält, ist insofern problematisch, als die sorgfältig abgestimmte Erzeugung und Ausdehnung der Aerosolwolke in Einklang gebracht werden muss mit der Einbringung des Aerosols in die Beatmungsluft.

Aus US 4.951.661 A ist dazu eine Anschlussvorrichtung bekannt, die ein T-förmiges Rohrelement umfasst, dass in die zu dem Patienten führende Beatmungsluftleitung einer Beatmungsvorrichtung eingesetzt wird. An dem Rohrstück, das senkrecht in das in die Beatmungsluftleitung einsetzbare Rohrstück einmündet, ist ein Aerosolgenerator vorgesehen, der eine Aerosolwolke erzeugt, die über das einmündende Rohrstück in die Beatmungsluft gelangt. Die Gestaltung des T-Stücks ist strömungstechnisch nicht optimal, was zu erheblichen Aerosol- und damit zu Medikamentverlusten führt. In DE 103 20 143 A werden ebenfalls T-förmige Anschlüsse an eine Beatmungsleitung vorgeschlagen, über die ein von einem Aerosolerzeuger bereitgestelltes Aerosol in die Beatmungsleitung eingebracht wird. Die aus DE 103 20 143 A bekannten T-Stücke sind aber strömungstechnisch wesentlich effektiver gestaltet, da praktisch der gesamte Querschnitt des in die Beatmungsleitung einmündenden Rohrstücks für die Zuleitung des Aerosols zur Verfügung steht.

Dennoch treten im Zusammenhang mit den bekannten T-Stücken für den Anschluss eines Inhalationstherapiegeräts an eine Beatmungsluftleitung eines Beatmungsgeräts immer wieder Aerosolverluste durch Niederschlagen von Aerosoltröpfchen/- partikeln an verschiedenen Oberflächen auf.

Vor diesem Hintergrund besteht die von der vorliegenden Erfindung zu lösende Aufgabe darin, eine Inhalationstherapievorrichtung anzugeben, bei der das erzeugte Aerosol und ein gasförmiges Medium, insbesondere Luft auf vorteilhafte Weise zusammengebracht und einem Patienten zum Einatmen dargeboten werden.

Eine weitere Aufgabe der vorliegenden Erfindung besteht darin, eine Inhalationstherapievorrichtung anzugeben, die sich in besonderer Weise für den Einsatz im Zusammenhang mit einer Beatmungsvorrichtung eignet.

Diese und weitere Aufgaben werden gelöst durch eine Inhalationsvorrichtung mit den Merkmalen gemäß Anspruch 1. Vorteilhafte Ausgestaltungen ergeben sich aus den Unteransprüchen.

Danach umfasst eine erfindungsgemäße Inhalationstherapievorrichtung in einer bevorzugten Ausführung eine Aerosolerzeugungseinrichtung, die für das Erzeugen eines Aerosols aus einer medikamenthaltigen Flüssigkeit und für die Abgabe des Aerosols in einen Ausdehnungsraumbereich ausgelegt ist, eine Flüssigkeitsbevorratungseinrichtung, die für die Bevorratung der Flüssigkeit und für die Zuführung der Flüssigkeit zu der Aerosolerzeugungseinrichtung ausgebildet ist, eine Zuführungseinrichtung, die für die Zuführung eines gasförmigen Mediums insbesondere Luft ausgebildet ist, und ein zylindrischen Rohrstück, das um den Ausdehnungsraumbereich derart angeordnet ist, dass das zugeführte gasförmige Medium auf die äußere Manteloberfläche des Rohrstücks auftrifft und an einem stirnseitigen Ende in das Innere des Rohrstücks strömt.

Durch das Rohrstück wird auf effektive Weise eine Ab- und Umlenkung der Strömung des zugeführten gasförmigen Mediums erreicht. Über den Rand des stirnseitigen Endes tritt das gasförmige Medium in den Bereich ein, in dem sich die Aerosolwolke ausdehnt, die von dem Aerosolgenerator aus der bevorrateten Flüssigkeit erzeugt wird.

In einer verallgemeinerten Betrachtungsweise umfasst eine erfindungsgemäße Inhalationstherapievorrichtung neben der bereits zuvor erwähnten Aerosolerzeugungseinrichtung, Flüssigkeitsbevorratungseinrichtung und Zuführungseinrichtung eine Strömungsbeeinflussungseinrichtung, die den Strömungsverlauf des zugeführten gasförmigen Mediums beeinflussend derart ausgebildet ist, dass das gasförmige Medium einen Mantelstrom um den Ausdehnungsraumbereich für das Aerosol ausbildet.

Die Mantelströmung ist dabei als quasi-laminare Strömung des gasförmigen Mediums in den Bereichen am Rand des Ausdehnungsraumbereich für die Aerosolwolke zu verstehen.

Im Folgenden wird die Erfindung anhand von Ausführungsbeispielen unter Bezugnahme auf die beigefügten Zeichnungen genauer beschrieben. In den Zeichnungen zeigt:
- Figur 1: eine geschnittene Seitenansicht eines ersten Ausführungsbeispiels einer erfindungsgemäßen Inhalationstherapievorrichtung; und
- Figur 2: eine geschnittene Draufsicht auf das erste Ausführungsbeispiel einer erfindungsgemäßen Inhalationstherapievorrichtung.

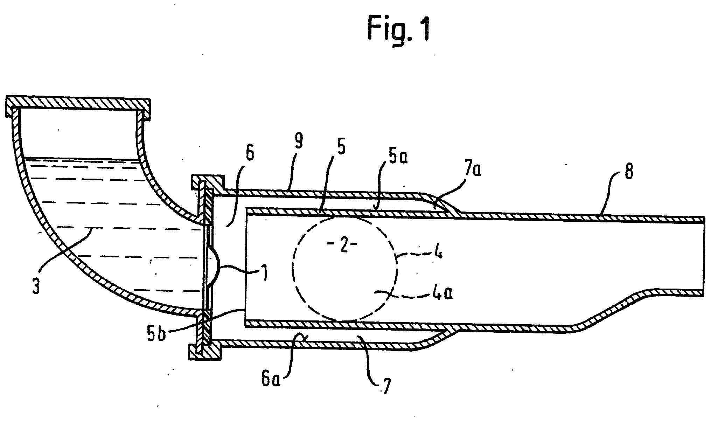

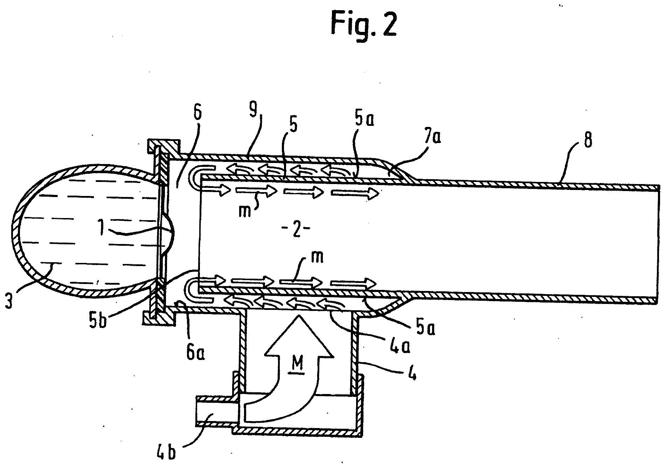

Im Folgenden wird ein Ausführungsbeispiel einer Inhalationstherapievorrichtung gemäß der Erfindung anhand der Figuren 1 und 2 erläutert, die beide das erste Ausführungsbeispiel in einer geschnittenen Darstellung jedoch aus unterschiedlichen Blickrichtungen zeigen.

In den Figuren 1 und 2 ist eine erfindungsgemäße Inhalationstherapievorrichtung dargestellt, die eine Aerosolerzeugungseinrichtung 1, beispielsweise einen Membran-Aerosolgenerator aufweist. Die Aerosolerzeugungseinrichtung 1 erzeugt aus einer medikamenthaltigen Flüssigkeit oder Fluid ein Aerosol, das sie in einen Ausdehnungsraumbereich 2 hinein abgibt, in dem sich das erzeugte Aerosol ausdehnen kann. Insbesondere bei Membran-Aerosolgeneratoren erfolgt die Aerosolerzeugung und Abgabe in Form einer sich in gewissem Umfang gerichtet ausdehnenden Aerosolwolke, die sich vom Aerosolgenerator 1 aus in den Ausdehnungsbereich 2 hinein ausdehnt.

Die zu vernebelnde Flüssigkeit wird in einer Flüssigkeitsbevorratungseinrichtung 3, beispielsweise einem Flüssigkeitsreservoir bevorratet und der Aerosolerzeugungseinrichtung 1 zugeführt. An dieser Stelle sei angemerkt, dass abweichend von dem in den Figuren 1 und 2 gezeigten Ausführungsbeispiel einer erfindungsgemäßen Inhalationstherapievorrichtung die Bevorratung und die Zuführung der zu vernebelnden Flüssigkeit durch getrennte Einrichtungen erfolgen kann; jedoch wird die in den Figuren 1 und 2 gezeigte Flüssigkeitsbevorratungseinrichtung 3 bevorzugt, die gleichzeitig der Bevorratung und Zuführung der Flüssigkeit dient.

Das Ausführungsbeispiel einer erfindungsgemäßen Inhalationstherapievorrichtung umfasst ferner eine Zuführungseinrichtung 4 für die Zuführung eines gasförmigen Mediums, bei dem es sich insbesondere um Luft, ein therapeutisch wirksames Gas oder ein für die Diagnostik geeignetes Gas handelt. Über die Zuführungseinrichtung 4 wird der erfindungsgemäßen Inhalationstherapievorrichtung das gasförmige Medium zugeführt, so dass es im Rahmen der Inhalationstherapie von einem Patienten eingeatmet werden kann. Die Zuführungseinrichtung 4 eignet sich in einer besonders bevorzugten Ausgestaltung des ersten Ausführungsbeispiels für die Zuführung der Beatmungsluft einer Beatmungsvorrichtung (nicht dargestellt), die an dieser Stelle nicht weiter erläutert werden soll, die aber dem in diesem Bereich tätigen Fachmann grundsätzlich bekannt ist. Jedoch ist für die Auslegung der erfindungsgemäßen Zuführungseinrichtung 4 in dieser besonderen Ausgestaltung zu beachten, dass dabei die Zuführungseinrichtung 4 vorzugsweise für die Zuführung der gesamten Atemluft ausgelegt ist, so dass über die zugeführte Beatmungsluft dem Patienten die für die Atmung erforderliche Luft über die Zuführungseinrichtung 4 der erfindungsgemäßen Inhalationstherapievorrichtung zugeführt werden kann. Auf diese Weise ist es möglich mit Hilfe einer erfindungsgemäßen Inhalationstherapievorrichtung gemäß dem ersten Ausführungsbeispiel den Patienten zu beatmen oder dem Patienten ein Atemmuster vorzugeben, da die gesamte für die Atmung bereitgestellte Luft über die Zuführungseinrichtung 4 zugeführt wird. Sofern keine Beatmungsluft aus einem Beatmungsgerät über die Zuführeinrichtung 4 zugeführt wird, kann die Zuführungseinrichtung 4 für die Zuführung von Umgebungsluft ausgelegt werden, beispielsweise indem sie sich zur Umgebung hin öffnet. Die zuvor im Hinblick auf eine Beatmungsvorrichtung erläuterte aktive Zuführung des gasförmigen Mediums über die Zuführungseinrichtung 4 ist aber die bevorzugte Ausgestaltung im Rahmen der Erfindung.

Das über die Zuführungseinrichtung 4 zugeführte gasförmige Medium trifft bei dem in den Figuren 1 und 2 gezeigten Ausführungsbeispiel einer Inhalationstherapievorrichtung gemäß der vorliegenden Erfindung auf eine Strömungsbeeinflussungseinrichtung 5. Der Strömungsverlauf des zugeführten gasförmigen Mediums M wird durch die Strömungsbeeinflussungseinrichtung 5 derart beeinflusst, dass das gasförmige Medium M einen Mantelstrom m um den Ausdehnungsbereich 2 für das Aerosol ausbildet. Der Strömungsverlauf des zugeführten gasförmigen Mediums M ist in Figur 2 in der Zeichnungsebene beispielhaft angedeutet. Dazu zeigt Figur 2 mehrere Pfeile, die das zugeführte gasförmige Medium M repräsentieren, das sich um die Strömungsbeeinflussungseinrichtung 5 ausbreitet und die Strömungsbeeinflussungseinrichtung 5 so umströmt, dass es schließlich am Rand des Ausdehnungsraumbereichs 2 entlang strömt, in.dem sich zentral die von dem Aerosolgenerator 1 abgegebene Aerosolwolke ausbreitet. Durch die Gestaltung der Strömungsbeeinflussungseinrichtung 5 wird, wie Figur 2 zeigt, erreicht, dass das gasförmige Medium M sich im wesentlichen am Rand des Ausdehnungsbereichs 2 in Form einer Mantelströmung m ausbildet, die als quasi-laminare Strömung in den Randbereichen des Ausdehnungsbereichs 2 angesehen werden kann. Zwar findet eine durch in der Praxis unvermeidbare geringfügige Turbulenzen verursachte Vermischung der Aerosolwolke und der Mantelströmung des gasförmigen Mediums beim Fortschreiten beider durch den Ausdehnungsraumbereich 2 statt, jedoch ist aufgrund der Ab- und Umlenkung des zugeführten gasförmigen Mediums M durch die Strömungsbeeinflussungseinrichtung 5 gewährleistet, dass sich in erheblichen Maße das gasförmige Medium zunächst in Form einer Mantelströmung m um die Aerosolwolke legt, die sich ausdehnend im Ausdehnungsraumbereich 2 vom Aerosolgenerator 1 weg bewegt, ebenso wie die Mantelströmung m des zugeführten gasförmigen Mediums M.

Bei dem in den Figuren 1 und 2 gezeigten Ausführungsbeispiel der Erfindung ist die Strömungsbeeinflussungseinrichtung in Form eines zylindrischen Rohrstücks 5 realisiert, das um den Ausdehnungsraumbereich 2 angeordnet ist. Die vom Aerosolgenerator erzeugte Aerosolwolke dehnt sich in das zylindrische Rohrstück 5 hinein aus. Wie sich aus den Figuren 1 und 2 ferner ergibt, ist das zylindrische Rohrstück bei dem gezeigten Ausführungsbeispiel im Querschnitt kreisförmig, was zweckdienlich und vorteilhaft ist. Auf die äußere Manteloberfläche 5a des Rohstücks 5 trifft das zugeführte gasförmige Medium M auf. Es strömt entlang der äußeren Manteloberfläche 5a um das zylindrische Rohrstück 5 herum und dehnt sich dabei in Richtung auf das stirnseitige Ende 5b des Rohrstücks 5 aus. Durch die Ab- und Umlenkung der Strömung des gasförmigen Mediums M wird das gasförmige Medium M im wesentlichen gleichmäßig um das zylindrische Rohrstück 5 herum verteilt und erreicht das stirnseitige Ende 5b, das dem Aerosolgenerator 1 gegenüber angeordnet ist, so dass das gasförmige Medium um das stirnseitige Ende 5b des Rohrstücks 5 herum strömt und im Randbereich der Ausdehnungszone 2 der Aerosolwolke entlang der inneren Oberfläche des Rohrstücks 5 weiterströmt. Dadurch bildet sich die angestrebte zylindrische Mantelströmung m um den Ausdehnungsraumbereich 2 für das Aerosol aus. Beim Fortschreiten der Mantelströmung m und der Aerosolwolke durch das Rohrstück 5 tritt eine kontinuierliche aber behutsame Vermischung des gasförmigen Mediums und der Aerosolwolke auf, da aufgrund des quasilaminaren Charakters der Mantelströmung m keine starken Turbulenzen auftreten. Beim Fortschreiten der sich ausdehnenden Aerosolwolke ist durch die Mantelströmung m dabei aber gewährleistet, dass ein Niederschlagen der Aerosoltröpfchen/-partikel an der inneren Wandoberfläche des Rohrstücks 5 praktisch nicht auftritt.

Wie in den Figuren 1 und 2 dargestellt, ist bei dem gezeigten Ausführungsbeispiel vorteilhafterweise das Rohrstück 5 einstückig mit einem Mundstück 8 oder einem Anschlussstutzen für eine Atemmaske ausgebildet, über das der Patient das Aerosol und das zugeführte gasförmige Medium einatmet. Im Aufbau ähnelt ein Anschlussstutzen für eine Atemmaske dem in der Figur gezeigen Mundstück 8, so dass eine zusätzliche Darstellung entbehrlich ist und auch im Hinblick auf den Anschlussstutzen für eine Atemmaske auf Figuren 1 und 2 Bezug genommen werden kann. Im Falle der Zuführung der Beatmungsluft eines Beatmungsgeräts wird dem Patienten über das Mundstück 8 / die Atemmaske die gesamte Atemluft zur Verfügung gestellt. Durch die einstückige Ausgestaltung des Rohrstücks 5 und des Mundstücks 8 / den Anschlussstutzen wird erreicht, dass ein Niederschlagen der Aerosoltröpfchen/- partikel verhindernde Mantelströmung m praktisch bis zum Eintritt in den Mund- und Rachenraum des Patienten aufrecht erhalten bleibt.

Bei dem in den Figuren 1 und 2 gezeigten Ausführungsbeispiel einer erfindungsgemäßen Inhalationstherapievorrichtung ist das zylindrische Rohrstück 5 in einer Kammer 6 angeordnet, in die hinein der Aerosolerzeuger 1 das Aerosol abgibt. Die Kammer 6 wird umschlossen von einem Abschnitt 9 des Gehäuses der Inhalationstherapievorrichtung, wobei der Gehäuseabschnitt 9, ähnlich wie das zylindrische Rohrstück 5, einen zylindrischen, beispielsweise einen kreiszylindrischen Querschnitt aufweist. Diese Abstimmung zwischen Kammer 6 und Rohrstück 5 ist aber nicht zwingend erforderlich, um den hier erläuterten Effekt zu erzielen. Denn in jedem Fall befindet sich zwischen der Innenwand 6a der Kammer 6 und der äußeren Manteloberfläche 5a des zylindrischen Rohrstücks 5 ein Spaltraum 7, in dem sich das zugeführte gasförmige Medium M ausbreiten kann.

Da der Spaltraum 7 in einem vom Aerosolerzeuger 1 entfernt angeordneten Bereich 7a abgeschlossen ist, findet die Ausbreitung des gasförmigen Mediums M in Richtung auf das stirnseitige Ende 5b des Rohrstücks 5 statt, das dem Aerosolgenerator 1 gegenüberliegt. Der Abschluss des Spaltraums 7 im Bereich 7a erfolgt vorzugsweise derart, dass das Gehäuse 9 der Inhalationstherapievorrichtung einstückig mit dem Rohrstück 5 ausgebildet wird, wie in den Figuren 1 und 2 für das erläuterte Ausführungsbeispiel gezeigt ist.

Aus den Figuren 1 und 2 ergibt sich für das erläuterte Ausführungsbeispiel der Inhalationstherapievorrichtung gemäß der Erfindung ferner, dass die Zuführeinrichtung für das gasförmige Medium M vorzugsweise ein zylindrischer Anschlussstutzen 4 ist, der an dem Gehäuse 9 der Inhalationstherapievorrichtung vorgesehen ist und sich zur Kammer 6 hin öffnet. Die Austrittsöffnung 4a des zylindrischen Anschlussstutzens 4 ist dabei so angeordnet, dass sie auf das Rohrstück 5 ausgerichtet ist, so dass dieses ab- und umlenkend auf das gasförmige Medium M einwirkt.

In Figur 1 ist die Lage der Austrittsöffnung 4a bzw. des zylindrischen Anschlussstutzens 4 durch eine gestrichelte Linie angedeutet. Aus dieser Darstellung ergibt sich, dass der zylindrische Anschlussstutzen 4 vorzugsweise einen kreiszylindrischen Querschnitt aufweist und mittig zu dem zylindrischen Rohrstück 5 angeordnet ist. Jedoch kann der Anschlussstutzen 4 auch mit einem anderen Querschnitt, beispielsweise einem elliptischen Querschnitt ausgestattet und/oder außermittig zu dem Rohrstück 5 angeordnet sein. Erfindungsgemäß entscheidend ist, dass die Zuführung des gasförmigen Mediums M mittels der Zuführeinrichtung 4 in Bezug auf die Strömungsbeeinflussungseinrichtung 5 so erfolgt, das letztere ihre ab- und umlenkende Wirkung auf das zugeführte gasförmige Medium M entfaltet und eine Mantelströmung m um den Ausdehnungsraumbereich 2 für das Aerosol hervorgerufen wird.

Die Zuführeinrichtung 4 weist des gezeigten Ausführungsbeispiels weist ferner ein Anschlusselement 4b für den Anschluss einer Zuleitung, beispielsweise eines Schlauchs auf. Über den Schlauch wird das gasförmige Medium M insbesondere die Beatmungsluft von einem Beatmungsgerät der erfindungsgemäßen Inhalationstherapievorrichtung zugeführt. Letztlich erlaubt die Zuführungseinrichtung 4 ebenso, dasss andersartig erzeugte Luftströmungen zugeführt werden können, beispielsweise auch die durch einen aktiv atmenden Patienten angesaugte Umgebungsluft, die in die Zuführungseinrichtung 4 eintritt.

## Patentansprüche

1. Inhalationstherapievorrichtung, mit:
- einer Aerosolerzeugungseinrichtung (1), die für das Erzeugen eines Aerosols aus einer medikamenthaltigen Flüssigkeit und für die Abgabe des Aerosols in einen Ausdehnungsraumbereich (2) ausgelegt ist,
- einer Zuführungseinrichtung (4), die für die Zuführung eines gasförmigen Mediums (M) insbesondere Luft ausgebildet ist,
- einer Strömungsbeeinflussungseinrichtung (5), die den Strömungsverlauf des zugeführten gasförmigen Mediums (M) derart beeinflussend ausgebildet ist, dass an einem Rohrstück ein Ab- und Umlenken der Strömung des zugeführten gasförmigen Mediums erreicht wird und das zugeführte gasförmige Medium über einen Rand eines stirnseitigen Endes des Rohstücks in einen Bereich des Rohrstücks eintritt, in dem sich das Aerosol ausdehnt, und entlang einer inneren Oberfläche des Rohrstücks weiterströmt,und
wobei die Zuführungseinrichtung (4) ferner für die Zufuhr der gesamten Atemluft eines Patienten ausgebildet ist.

2. Inhalationstherapievorrichtung nach Anspruch 1, wobei der Patient über die Zuführungseinrichtung (4) beatmet oder dem Patienten über die Zuführungseinrichtung (4) ein Atemmuster vorgegeben werden kann.

3. Inhalationstherapievorrichtung nach Anspruch 1 oder 2, wobei die Strömungsbeeinflussungseinrichtung (5) den Strömungsverlauf des zugeführten gasförmigen Mediums (M) ferner derart beeinflussend ausgebildet ist, dass das gasförmige Medium einen Mantelstrom (m) am Rand des Ausdehnungsraumbereichs (2) für das Aerosol ausbildet.

4. Inhalationstherapievorrichtung nach Anspruch 1 oder 2, wobei die Strömungsbeeinflussungseinrichtung (5) den Strömungsverlauf des zugeführten gasförmigen Mediums (M) ferner derart beeinflussend ausgebildet ist, dass aufgrund der Ab- und Umlenkung das gasförmige Medium sich zunächst in Form einer Mantelströmung (m) um die Aerosolwolke legt, bevor eine Vermischung der Aerosolwolke und der Mantelströmung beim Fortschreiten beider durch den Ausdehnungsbereich (2) stattfindet.

5. Inhalationstherapievorrichtung nach einem der vorhergehenden Ansprüche, ferner mit:
- einem Flüssigkeitsbevorratungseinrichtung (3), die für die Bevorratung der Flüssigkeit und für die Zuführung der Flüssigkeit zu der Aerosolerzeugungseinrichtung (1) ausgebildet ist,
wobei
das Rohrstück (5) ein zylindrisches Rohrstück ist und um den Ausdehnungsraumbereich (2) derart angeordnet ist, dass das zugeführte gasförmige Medium (M) auf die äußere Manteloberfläche (5a) des Rohrstücks (5) auftrifft und an einem stirnseitigen Ende (5b) in das Innere des Rohrstücks (5) strömt.

6. Inhalationstherapievorrichtung nach Anspruch 5, wobei das zylindrische Rohrstück (5) in einer von der Inhalationstherapievorrichtung umschlossenen Kammer (6) derart angeordnet ist, dass zwischen einer äußeren Manteloberfläche (5a) des zylindrischen Rohrstücks (5) und einer inneren Wandoberfläche (6a) der Kammer (6) ein Spaltraum (7) für die Ausbreitung des zugeführten gasförmigen Mediums (M) ausgebildet ist.

7. Inhalationstherapievorrichtung nach Anspruch 5 oder 6, wobei das stirnseitiges Ende (5b) des zylindrischen Rohrstücks (5) vor der Aerosolerzeugungseinrichtung (1) angeordnet ist.

8. Inhalationstherapievorrichtung nach Anspruch 7, **dadurch gekennzeichnet, dass** der Abstand zwischen dem stirnseitigen Ende (5b) des zylindrischen Rohrstücks (5) und der Aerosolerzeugungseinrichtung (1) kleiner als der größte Durchmesser des Rohrstücks (5) ist.

9. Inhalationstherapievorrichtung nach einem der vorhergehenden Ansprüche, wobei das zylindrische Rohrstück (5) kreiszylinderförmig ist.

10. Inhalationstherapievorrichtung nach einem der vorhergehenden Ansprüche, wobei ferner ein Mundstück (8) oder ein Anschlussstutzen für eine Atemmaske vorgesehen ist, das einstückig mit dem zylindrischen Rohrstück (5) ausgebildet ist.

11. Inhalationstherapievorrichtung nach Anspruch 3 oder einem der Ansprüche 6 bis 10, wobei ein die Kammer (6) umschließender Gehäuseabschnitt (9) der Inhalationstherapievorrichtung einstückig mit dem zylindrischen Rohrstück (5) ausgebildet ist.

12. Inhalationstherapievorrichtung nach einem der vorhergehenden Ansprüche, wobei die Zuführeinrichtung für das gasförmige Medium (M) ein zylindrischer Anschlussstutzen (4) ist.

13. Inhalationstherapievorrichtung nach Anspruch 12, wobei der zylindrische Anschlussstutzen (4) eine zur Strömungsbeeinflussungseinrichtung (5) gerichtete Austrittsöffnung (4a) aufweist.

14. Inhalationstherapievorrichtung nach einem der vorhergehenden Ansprüche, wobei die Zuführeinrichtung für das gasförmige Medium (M) eine Anschlusseinrichtung (4b) für den Anschluss einer Zuführleitung für das gasförmige Medium (M) aufweist.

15. Inhalationstherapievorrichtung nach einem der vorhergehenden Ansprüche, wobei die Zuführeinrichtung (4) ausgebildet ist für die Zuführung von Beatmungsluft einer Beatmungsvorrichtung.

## Claims

1. Inhalation therapy device having:
- an aerosol-generating device (1) which is designed to generate an aerosol from a medicament-containing liquid and to release the aerosol into an expansion chamber area (2),
- a supply device (4) which is designed to supply a gaseous medium (M), in particular air,
- a flow-influencing device (5) which is designed to influence the flow path of the supplied gaseous medium (M) such that deflection and diversion of the flow of the supplied gaseous medium is achieved at a pipe section and the supplied gaseous medium enters an area of the pipe section in which the aerosol is expanded via an edge of an end-face side end of the pipe section, and flows further along an inner surface of the pipe section, and
wherein the supply device (4) is further designed to supply the entire respiratory air of a patient.

2. Inhalation therapy device according to claim 1, wherein the patient is artificially respirated via the supply device (4) or a respiratory pattern may be preset for the patient via the supply device (4).

3. Inhalation therapy device according to claim 1 or 2, wherein the flow-influencing device (5) is designed to further influence the flow path of the supplied gaseous medium (M) such that the gaseous medium forms a by-pass flow (m) at the edge of the expansion chamber area (2) for the aerosol.

4. Inhalation therapy device according to claim 1 or 2, wherein the flow-influencing device (5) is designed to further influence the flow path of the supplied gaseous medium (M) such that due to deflection and diversion, the gaseous medium is first of all settled around the aerosol cloud in the form of a by-pass flow (m) before mixing of the aerosol cloud and the by-pass flow takes place during progression of both through the expansion area (2).

5. Inhalation therapy device according to one of the preceding claims, further having:
- a liquid storage device (3) which is designed to store the liquid and to supply the liquid to the aerosol-generating device (1),
wherein
the pipe section (5) is a cylindrical pipe section and is arranged around the expansion chamber area (2) such that the supplied gaseous medium (M) meets the outer by-pass surface (5a) of the pipe section (5) and flows past an end-face side end (5b) into the interior of the pipe section (5).

6. Inhalation therapy device according to claim 5, wherein the cylindrical pipe section (5) is arranged in a chamber (6) surrounded by the inhalation therapy device such that a splitting chamber (7) for the dispersion of the supplied gaseous medium (M) is formed between an outer by-pass surface (5a) of the cylindrical pipe section (5) and an inner wall surface (6a) of the chamber (6).

7. Inhalation therapy device according to claim 5 or 6, wherein the end-face side end (5b) of the cylindrical pipe section (5) is arranged upstream of the aerosol-generating device (1).

8. Inhalation therapy device according to claim 7, **characterised in that** the distance between the end-face side end (5b) of the cylindrical pipe section (5) and the aerosol-generating device (1) is less than the greatest diameter of the pipe section (5).

9. Inhalation therapy device according to one of the preceding claims, wherein the cylindrical pipe section (5) is like a circular cylinder.

10. Inhalation therapy device according to one of the preceding claims, wherein further a mouthpiece (8) or a connecting piece for a breathing mask is provided which is designed to be integral with the cylindrical pipe section (5).

11. Inhalation therapy device according to claim 3 or one of claims 6 to 10, wherein a housing section (9) of the inhalation therapy device surrounding the chamber (6) is designed to be integral with the cylindrical pipe section (5).

12. Inhalation therapy device according to one of the preceding claims, wherein the supply device for the gaseous medium (M) is a cylindrical connecting piece (4).

13. Inhalation therapy device according to claim 12, wherein the cylindrical connecting piece (4) has an outlet opening (4a) directed to the flow-influencing device (5).

14. Inhalation therapy device according to one of the preceding claims, wherein the supply device for the gaseous medium (M) has a connecting device (4b) to connect a supply pipe for the gaseous medium (M).

15. Inhalation therapy device according to one of the preceding claims, wherein the supply device (4) is designed to supply respiratory air to a respirator.

## Revendications

1. Appareil de thérapie par inhalation avec :
- un dispositif producteur d'aérosol (1), qui est conçu pour produire un aérosol à partir d'un liquide contenant un médicament et pour délivrer l'aérosol dans une zone d'espace de dilatation (2),
- un dispositif d'alimentation (4), qui est réalisé pour amener un fluide sous forme gazeuse (M), en particulier de l'air, et
- un dispositif influençant l'écoulement (5), qui est réalisé de manière à influencer l'écoulement du fluide sous forme gazeuse (M) amené de telle sorte qu'un détournement et une déviation de l'écoulement du fluide sous forme gazeuse amené sont obtenus au niveau d'une pièce tubulaire et que le fluide sous forme gazeuse amené entre par l'intermédiaire d'un bord d'une extrémité frontale de la pièce tubulaire dans une zone de la pièce tubulaire dans laquelle se dilate l'aérosol et s'écoule ensuite le long d'une surface intérieure de la pièce tubulaire, et
dans lequel le dispositif d'alimentation (4) est réalisé en plus pour amener la totalité de l'air de respiration d'un patient.

2. Appareil de thérapie par inhalation selon la revendication 1, dans lequel le patient respire par l'intermédiaire du dispositif d'alimentation (4) ou un modèle respiratoire peut être prescrit au patient par l'intermédiaire du dispositif d'alimentation (4).

3. Appareil de thérapie par inhalation selon la revendication 1 ou 2, dans lequel le dispositif influençant l'écoulement (5) est réalisé de manière à influencer en plus l'écoulement du fluide sous forme gazeuse (M) amené de telle sorte que le fluide sous forme gazeuse réalise un courant enveloppant (m) au niveau du bord de la zone d'espace de dilatation (2) pour l'aérosol.

4. Appareil de thérapie par inhalation selon la revendication 1 ou 2, dans lequel le dispositif influençant l'écoulement (5) est réalisé de manière à influencer en plus l'écoulement du fluide sous forme gazeuse (M) amené de telle sorte que, en raison du détournement et de la déviation, le fluide sous forme gazeuse se trouve d'abord sous la forme d'un courant enveloppant (m) autour du nuage d'aérosol avant qu'un mélange du nuage d'aérosol et du courant enveloppant ait lieu lors de la progression de ces deux fluides à travers la zone de dilatation (2).

5. Appareil de thérapie par inhalation selon l'une des revendications précédentes, avec en plus
- un dispositif d'approvisionnement en liquide (3), qui est réalisé pour l'approvisionnement en liquide et pour amener un liquide au dispositif producteur d'aérosol (1),
dans lequel
la pièce tubulaire (5) est une pièce tubulaire cylindrique et est agencée de telle manière autour de la zone d'espace de dilatation (2) que le fluide sous forme gazeuse (M) amené arrive sur la surface enveloppante extérieure (5a) de la pièce tubulaire (5) et s'écoule dans l'intérieur de la pièce tubulaire (5) au niveau d'une extrémité frontale (5b).

6. Appareil de thérapie par inhalation selon la revendication 5, dans lequel la pièce tubulaire cylindrique (5) est agencée de telle manière dans une chambre (6) entourée par l'appareil de thérapie par inhalation qu'un espace en fente (7) pour la diffusion du fluide sous forme gazeuse (M) amené est réalisé entre une surface enveloppante extérieure (5a) de la pièce tubulaire cylindrique (5) et une surface de paroi intérieure (6a) de la chambre (6).

7. Appareil de thérapie par inhalation selon la revendication 5 ou 6, dans lequel l'extrémité frontale (5b) de la pièce tubulaire cylindrique (5) est agencée devant le dispositif producteur d'aérosol (1).

8. Appareil de thérapie par inhalation selon la revendication 7, **caractérisé en ce que** la distance entre l'extrémité frontale (5b) de la pièce tubulaire cylindrique (5) et le dispositif producteur d'aérosol (1) est plus petite que le plus grand diamètre de la pièce tubulaire (5).

9. Appareil de thérapie par inhalation selon l'une des revendications précédentes, dans lequel la pièce tubulaire cylindrique (5) est de forme cylindrique circulaire.

10. Appareil de thérapie par inhalation selon l'une des revendications précédentes, dans lequel une embouchure (8) ou un embout de raccordement destiné(e) à un masque respiratoire est prévu(e) en plus et est réalisé(e) d'un seul tenant avec la pièce tubulaire cylindrique (5).

11. Appareil de thérapie par inhalation selon la revendication 3 ou l'une des revendications 6 à 10, dans lequel une partie de boîte (9) entourant la chambre (6) de l'appareil de thérapie par inhalation est réalisée d'un seul tenant avec la pièce tubulaire cylindrique (5).

12. Appareil de thérapie par inhalation selon l'une des revendications précédentes, dans lequel le dispositif d'alimentation pour le fluide sous forme gazeuse (M) est un embout de raccordement cylindrique (4).

13. Appareil de thérapie par inhalation selon la revendication 12, dans lequel l'embout de raccordement cylindrique (4) comporte un orifice de sortie (4a) orienté vers le dispositif influençant l'écoulement (5).

14. Appareil de thérapie par inhalation selon l'une des revendications précédentes, dans lequel le dispositif d'alimentation pour le fluide sous forme gazeuse (M) comporte un dispositif de raccordement (4b) destiné au raccordement d'une conduite d'alimentation pour le fluide sous forme gazeuse (M).

15. Appareil de thérapie par inhalation selon l'une des revendications précédentes, dans lequel le dispositif d'alimentation (4) est réalisé pour amener de l'air de respiration artificielle d'un appareil de respiration artificielle.
